(19) **Europäisches Patentamt European Patent Office Office européen des brevets**

(11) **EP 4 141 886 B1**

(12) **EUROPEAN PATENT SPECIFICATION**

(45) Date of publication and mention
of the grant of the patent:
**20.05.2026 Bulletin 2026/21**

(21) Application number: **21192600.1**

(22) Date of filing: **23.08.2021**

(51) International Patent Classification (IPC):
***G16H 50/30*** *(2018.01)*

(52) Cooperative Patent Classification (CPC):
**G16H 50/30**

(54) **METHOD AND SYSTEM AND APPARATUS FOR QUANTIFYING UNCERTAINTY FOR MEDICAL IMAGE ASSESSMENT**

**VERFAHREN UND SYSTEM UND VORRICHTUNG ZUR BESTIMMUNG DER UNSICHERHEIT BEI DER BEURTEILUNG MEDIZINISCHER BILDER**

**PROCÉDÉ ET SYSTÈME PERMETTANT DE QUANTIFIER L'INCERTITUDE POUR L'ÉVALUATION D'IMAGES MÉDICALES**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.03.2023 Bulletin 2023/09**

(73) Proprietor: **Siemens Healthineers AG**
**91301 Forchheim (DE)**

(72) Inventors:
* **GHESU, Florin-Cristian**
**Skillman, NJ 08558 (US)**
* **MANSOOR, Awais**
**Potomac, MD 20854 (US)**
* **GRBIC, Sasa**
**Plainsboro, NJ 08536 (US)**
* **VUNIKILI, Ramya**
**Secaucus, NJ 07094 (US)**
* **KUMAR KARN, Sanjeev**
**Plainsboro, NJ 08536 (US)**
* **AMBATI, Rajeev Bhatt**
**Plainsboro, NJ 08536 (US)**
* **FARRI, Oladimeji**
**Upper Saddle River, NJ 07458 (US)**
* **GEORGESCU, Bogdan**
**Princeton, NJ 08540 (US)**
* **COMANICIU, Dorin**
**Princeton, NJ 08540 (US)**

(74) Representative: **Schwarz, Claudia**
**Schwarz + Kollegen**
**Patentanwälte**
**Heilmannstraße 19**
**81479 München (DE)**

(56) References cited:
**US-A1- 2019 122 073**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to medical image processing and in particular to processing of uncertainties in medical health-related data, in particular in medical imaging data.

The document US 2019/0122073 A1 describes a method for uncertainty information propagation. A CT scan is Monte Carlo sampled, e.g., 50 times, to obtain a probability distribution of "statistics images". The "statistics images" are then merged/fused with the original CT scan image into a three channel CT image. The three channels correspond to the original CT scan along with the mean and variance images obtained from probabilistic segmentation output from a Bayesian neural network.

Generally, there do exist several different imaging modalities for acquiring medical images, like radiography, computed tomography (CT), magnetic resonance imaging (MRI), amongst others. The imaging procedure may be specifically adapted to image a particular organ or body part in order to find and/or assess clinical abnormalities and/or diseases and/or lesions. For example, for classifying pulmonary malignancies or pneumonia, typically, a computed tomography (CT) scan or chest radiograph (CXR) is executed.

The acquired medical images may be subject to an automated procedure for medical assessment, for example for initiating further measurements and/or the acquisition of further sensor data and/or the initiation of the clinical procedures. An automated procedure for assessing medical images is machine learning. The machine learning system may, for example, be configured for classifying between healthy tissue and a lesion (or an abnormality). Generally, a machine learning system may be configured for assessment of provided medical images.

However, the computer implemented and automatic assessment of medical images is subject to uncertainty, in particular aleatoric uncertainty. Aleatoric uncertainty is also known as statistical uncertainty, and is representative of unknowns that differ each time the same experiment is run. Aleatoric is derived from the Latin "alea" or "dice", referring to a game of chance. Aleatoric uncertainty is to be distinguished from epistemic uncertainty, which is a systematic uncertainty, and is due to things the system could in principle know or calculate but does not in practice. This may be because a measurement is not accurate or there is noise in a measurement or in the measured signal.

For example, the assessment of chest radiography (CXR) images, in particular in an outpatient setting, is an inherently ambiguous task. Internal studies reveal inter-rater agreement levels of 60-70% for the detection of e.g. lung nodules and 50-60 percent for the detection of consolidation/airspace opacity. This level of disagreement can often be attributed to the lack of clarity in deciding whether an abnormal region is indicating abnormality A (e.g., a lung nodule/mass) or abnormality B (e.g., consolidation). Current machine learning systems based solely on CXR assessment as well as evaluation studies are designed to force this decision, while in clinical practice, the radiologist would not make such decision and would document in the report the uncertainty between these two classes (most likely calling for a follow-up using another CXR or CT scan to achieve a clear answer). Also, machine learning systems for CXR assessment generally do not use any auxiliary non-imaging information to guide this decision (between abnormality A/B). This is different from the radiologist who would, e.g., use the fact that the patient in question has a fever to steer the decision towards abnormality B/consolidation, which is an effect of pneumonia/infection, which in turn explains the fever. This leads to systems that perform poorly/unexpectedly in such ambiguous cases, achieving limited performance and directly impacting the trust of the user.

Although more accurate than CXR to obtain relevant information (e.g., to be used subsequently or later for a differential diagnosis), similar ambiguities can be present in high-resolution chest CT. Radiologists often refer to additional information from Electronic Health Records (EHR), including but not limited to, reason for ordering the exam, history of patient illness and physical examinations, serological results, biomarkers from lab diagnostics diagnosis, etc. to gain clarity. A currently occurring common scenario is the differentiation of CoVID-19 in patients who are susceptible to respiratory conditions such as Interstitial Lung Disease (ILD) from those with underlying pulmonary malignancies.

Based on this, the object of the present invention is to provide means for improving the expressiveness and/or robustness of a machine learning system's result, based on imaging data and/or to make it possible to combine imaging data with non-imaging data to improve statements, which are deduced from the imaging data.

The object is achieved by the appended patent claims, in particular by a computer implemented method, and uncertainty quantifier, medical system and a computer program product.

Computer-implemented method for providing an uncertainty prediction for a medical assessment on imaging data, being provided by a machine-learning system, comprising the method steps of:

- Receiving a set of input data, comprising the imaging data, which have been provided to the machine-learning system and non-imaging data, each represented as a signal with some degree of noise, being quantified as uncertainty, in particular aleatoric uncertainty, wherein the non-imaging data comprise medical or healthcare data in a digital format or representation, which do not comprise image data acquired from an imaging modality;

- Providing an information fusion algorithm, wherein the information fusion algorithm is an algorithm for combining different data sets, provided in different formats, including the imaging data and the non-imaging data, wherein the information fusion algorithm uses an information fusion model and/or a graph neural network, being optimized for maximizing entropy in the non-imaging data;

- Applying the received set of input data on the provided information fusion algorithm, while modeling the propagation of uncertainty through the information fusion algorithm to predict an uncertainty for the medical assessment as a result , provided by the machine-learning system, based on the provided set of input data.

The term "non-imaging data" refers to medical or healthcare data in a digital format or representation, which do not comprise image data, acquired from an imaging modality. Non-imaging data may reflect non-imaging knowledge. Some of non-imaging data needs to be structured before further processing. As will be explained later in more detail, the present invention inter alia suggests using a graph neural network for data processing. In this respect, particular non-imaging data needs to be structured before passing to a graph neural network, e.g., EHR text. Thus, a preprocessing may be executed on non-imaging data. Preprocessing may include re-structuring data in a processable format (e.g., standardized and normalized to be processed in a graph neural network and/or an information fusion model) in a memory. Thus, the storing of the preprocessed data differs from the storing of the original non-imaging data (also referred to as signals).

"Noise" in this respect relates to signal or data portions, which do not comprise a payload signal. Noise may be quantified as uncertainty, in particular aleatoric or epistemic uncertainty. While aleatoric uncertainty is the most common, also a distributional uncertainty, and other types of uncertainty may be processed. In a preferred embodiment, deep representation learning, e.g., variational autoencoder, VAE, may be used and applied to encode the information to a compact representation, e.g., via the variational autoencoder and/or to denoise some of the collected input data. For more details with respect to the variational autoencoder it is referred to Kingma, D. P., & Welling, M. (2013). Auto-encoding variational bayes. arXiv preprint arXiv:1312.6114.

Input data are digital data or are transformed from analog signals to digital signals by means of a converter. Input data may comprise digital signals or more complex datasets, e.g., developments of signals over time. Input data may comprise imaging data, stemming from different imaging modalities and non-imaging data, like e.g., biomarkers, laboratory values, electronic healthcare records (EHR), measurement signals, like physiological signals, like blood pressure, body temperature, heart rate etc.

The information fusion algorithm is an algorithm for combining different data sets, provided in different formats, including e.g., imaging data and non-imaging data. The information fusion model does not need to be "deep" but can be. The information fusion model may be a deep fusion model optimized by mutual information criteria. The information fusion algorithm may be or may use (apply) an information fusion model and/or a graph neural network, being optimized for maximizing entropy in the non-imaging data. Usually, more than one non-imaging signal is used in order to improve quality of the uncertainty prediction. Preferably an information fusion model is used, where propagation of uncertainty is propagated through the model. Alternatively, or in addition, a graph neural network may be used. In still another embodiment, only a graph neural network may be used (without an information fusion model), wherein the graph neural network encompasses both the imaging data and the non-imaging data.

The result of the information fusion is an estimated or predicted uncertainty for the medical assessment. The uncertainty may be represented in a quantified form. The uncertainty may be based on a preconfigured metric. The uncertainty may be provided as a percentage.

The machine learning system is used to provide automatic assessment by taking into account imaging data. The machine learning system may for example be based on an artificial neural network, ANN, a deep neural network, DNN, a convolutional neural network, CNN, by using different learning algorithms, like reinforcement learning, supervised learning or semi-supervised learning or even unsupervised learning. Generally, machine learning models can serve to detect structures in an image, classify abnormalities in an image, etc.

According to a preferred embodiment of the present invention, the entropy of the information fusion model and/or the graph neural network, in particular the von Neumann entropy, is optimized by a greedy algorithm or by another optimization algorithm, e.g., dynamic programming, grid search, and/or divide and conquer techniques.

According to another preferred embodiment, the method may further comprise:

- Applying a selection algorithm for selecting a subset of provided input data, which minimizes a cost function and/or reduces uncertainty by using a reinforcement learning model.

According to another preferred embodiment, the input data of a set of input sources may be present or absent. The latter may be the case, if it turns out that providing the input data is too expensive (e.g., from a time/performance aspect, from a monetary aspect). Further, the method may provide suggestion result dataset, encoding a guided decision which of the absent input data sources would reduce uncertainty and/or would minimize a cost function. With this, the input data are prioritized with respect to reducing uncertainty and/or minimizing a cost function. The cost function may be pre-configurable via a user input on a user interface.

According to another preferred embodiment, providing input data of the set of input data sources may comprise measuring and/or acquiring data from imaging modalities and/or from medical databases (e.g., electronic health record, HER, lab values, radiology information system, RIS, picture archiving and communication system, PACS etc.).

According to another preferred embodiment, the non-imaging data comprises (but is not limited to) biomarkers, clinical notes, image annotations, medical report dictations, measurements, laboratory values, diagnostic codes, data from an EHR-database, and/or anamnestic data of the patient.

According to another preferred embodiment, a reinforcement learning model is based on a decision process, in particular a non-Markovian decision process

$$M = (S, A, T, R, \eta),$$

where S denotes a state space, A an action space, T a stochastic transition process, R a reward function and $\eta$ a discount factor, wherein actions represent providing additional input data sources.

According to another preferred embodiment, the reward function is defined to minimize the cost and/or to minimize the predicted uncertainty. Cost can be configured by the user in a configuration phase, e.g., cost can be financial and/or time/efficiency/performance-related, or other impairments.

According to another preferred embodiment, an uncertainty propagation model comprising a Bayesian deep model and/or Q-Learning and/or actor critic learning may be used for the reinforcement learning.

According to another preferred embodiment, an uncertainty propagation model, in particular a Bayesian deep model is used in the information fusion model.

According to another preferred embodiment, the information fusion model is capable of processing a situation, where a subset of input data sources is not available or only available by certain costs.

According to another preferred embodiment, the predicted uncertainty is patient-specific. Alternatively, or cumulatively, the predicted uncertainty may be imaging data specific. Alternatively, or cumulatively, the predicted uncertainty may be signal specific.

According to another preferred embodiment, on a user interface, a set of interaction buttons is provided so that a user can indicate that an input data source is not available during inference or that the action space of the non-Markovian decision process is limited to the data sources, being available so that the user may select the type of optimization and in particular if he or she wants to minimize prediction uncertainty or costs.

Up to now, the invention has been described with respect to the claimed method. Features, advantages or alternative embodiments herein can be assigned or transferred to the other claimed objects (e.g., the computer program or a device, i.e., the uncertainty quantifier or a computer program product) and vice versa. In other words, the apparatus or device can be improved with features described or claimed in the context of the method and vice versa. In this case, the functional features of the method are embodied by structural units of the apparatus or device or system and vice versa, respectively. Generally, in computer science a software implementation and a corresponding hardware implementation (e.g., as an embedded system) are equivalent. Thus, for example, a method step for "storing" data may be performed with a storage unit and respective instructions to write data into the storage. For the sake of avoiding redundancy, although the device may also be used in the alternative embodiments described with reference to the method, these embodiments are not explicitly described again for the device.

In another aspect the invention relates to an uncertainty quantifier for a medical assessment on imaging data, being provided by a machine-learning system, which is adapted to execute the method as described above.

In another aspect the invention relates to a medical system for a medical assessment on imaging data, being provided by a machine-learning system with a set of medical data sources and with an uncertainty quantifier as described above.

In another aspect the invention relates to a computer program product comprising program elements which induce a computer to execute the steps of the method for providing an uncertainty prediction for a machine-learning based medical assessment on imaging data according to any of the preceding method claims, when the program elements are loaded into a memory of the computer or are executed thereon.

In another aspect the invention relates to a computer program, the computer program being loadable into a memory unit of a computer system, including program code sections to make the computer system execute the method for providing an uncertainty prediction for a medical assessment on imaging data as described above, when the computer program is executed in said computer system.

In another aspect the invention relates to a computer-readable medium, on which program code sections of a computer program are stored or saved, said program code sections being loadable into and/or executable in a computing unit to make the computing unit execute the method for providing an uncertainty prediction for a medical assessment on imaging data as described above, when the program code sections are executed in the computing unit. The computing unit may comprise a processing unit.

The properties, features and advantages of this invention described above, as well as the manner they are achieved, become clearer and more understandable in the light of the following description and embodiments, which will be described in more detail in the context of the drawings. This following description does not limit the invention on the contained embodiments. Same components or parts can be labeled with the same reference signs in different figures. In general, the figures are not for scale.

These and other aspects of the invention will be apparent from and elucidated with reference to the embodiments described hereinafter.

BRIEF DESCRIPTION OF THE DRAWINGS

Fig. 1    is a structural block diagram showing a typical application scenario for a machine learning model in state of the art;

Fig. 2    is an overview of the structure and architecture of an uncertainty quantifier according to a preferred embodiment of the present invention;

Fig. 3    is a schematic representation of a graph neural network with minimal entropy and

Fig. 4    is another schematic representation of a graph neural network with maximum entropy;

Fig. 5    is a flow chart of a method according to a preferred embodiment of the present invention;

Fig. 6    is an exemplary processing of chest CXR image by the uncertainty quantifier for providing a result r.

DETAILED DESCRIPTION OF EMBODIMENTS

Current solutions for chest radiography assessment focus on image-level classification of findings without precise localization or provide approximate localization of findings without investigating the inherent uncertainty at instance level. The term "at instance level" relates to a particular location in the image. Several methods have been proposed for uncertainty quantification. However, they do not explicitly tackle this type of aleatoric uncertainty and/or natural class overlap. In addition, such methods do not use any non-imaging information in order to augment and improve the accuracy of the classification.

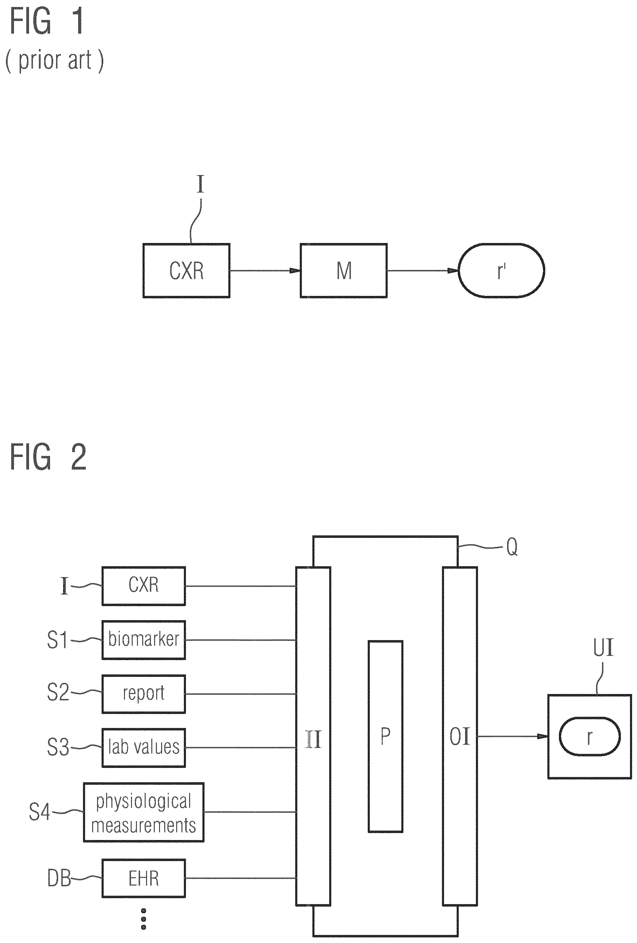

As can be seen in Fig. 1, a typical state of the art machine learning system, which is configured to processes input data in the form of imaging data I to provide a classification result r' without additional information with respect to quality and accuracy of the deduced classification. So, the user is not informed on how he can trust the result, provided by a machine learning model M.

Fig. 2 shows a schematic representation of a system with an uncertainty quantifier Q for providing the addition information, missing in prior art systems as mentioned above. As can be seen in Fig. 2, the uncertainty quantifier Q has an input interface II for receiving input data from a set or a variety of different sources, comprising image acquisition sources and non-image sources. The input data thus may comprise imaging data I, like e.g., chest radiography images, CXR, or computer tomography (CT) images or images form any other kind of image acquisition modalities. In addition, and as explained before, further non-imaging data are provided. The non-imaging data are referred to as signals S. For example, a biomarker signal S1, a clinical report signal S2, a set of laboratory signals S3, a set of physiological measurements S4, e.g., temperature, heart rate etc. Thus, the input interface II connects the uncertainty quantifier Q with a set of input data sources (not shown in the figure), like temperature sensor, heart rate sensor, a laboratory system etc. Alternatively, model in or cumulatively, databases, e.g., an electronic health record (EHR) may serve as input data source DB, too. The processor P is configured to implement and execute an image fusion algorithm. The result r of the image fusion algorithm is provided on an output interface OI. The result r is a prediction of (a quantified) uncertainty for a medical algorithmically calculated assessment, based on the received input data. The result r may be provided on a user interface UI. The user interface UI may also serve as human machine interface for receiving configuration data, provided by the user, e.g., for determining which of the input data sources is currently not or only available at high costs. This configuration data will be processed by the information fusion algorithm and/or by a selection algorithm.

In the example in Fig. 2 a CXR image is used as input. However, it is to be noted that this is only one of a set of examples and the invention is of course not restricted to this type of image modality. Thus, also MRI images, ultrasound images and images from other acquisition modalities may serve as input source.

The present invention provides a learning system that quantifies the predictive aleatoric uncertainty, e.g., a fuzzy prediction at instance level. There are two levels of ambiguity that can be quantified at this level:

1. Ambiguity between captured abnormality classes: this is the primary type of ambiguity that needs to be tackled. E.g., for nearly 15% of positive cases of nodule/consolidation in CXR a decision on the class cannot be performed based on the imaging information. This high degree of class-overlap is not unique to the mentioned, for instance, it can be found between pleural effusion and consolidation, or consolidation and lobal atelectasis, etc. One can model this type of ambiguity using different approaches for uncertainty quantification, including fuzzy predictions, evidential learning, subjective logic, etc. This leads to a system that is capable of accurately recognizing these 15% of cases by yielding multiple labels for the same instance of abnormality in the image.

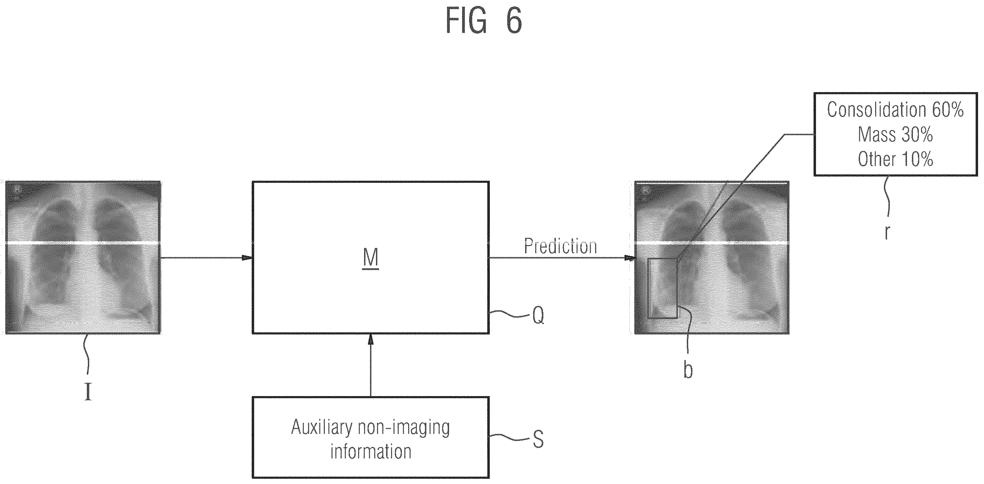

As can be seen in Figure 6, the highlighted region in the form of a bounding box b may refer to two potential abnormalities, a consolidation caused by an infection or a pulmonary mass. The result r is provided with a dataset comprising the indications "consolidation 60%, mass 30%, other 10%".

2. Out-of-training domain ambiguity: For the sake of completion, the second type of ambiguity is derived from whether the instance of abnormality is fully captured in the training distribution. Meaning, is there a chance that the instance may be part of a type of abnormality that was not modelled in the training and thus cannot be predicted by the system? As can be seen in Figure 1, the system recognizes that there is a non-zero chance that the bounding box may refer to something abnormal but is not part of the classes modelled in the training of the device.

According to a preferred embodiment, the behavior of the expert radiologist is emulated by using additional information from the non-imaging sources S to achieve more clarity when assessing such ambiguous cases. For the context of out-patient chest radiography assessment there are a series factors (based on non-imaging information) that can steer the decision of the radiologist in how to assess the case. In the concrete example in Figure 6, this would mean altering the decision, further increasing the probability of consolidation, for example. In practice, these factors may refer to "Patient's Age", "Indication of Fever", "Acute symptoms" or "Indication of Pain". This information may be provided with the order for exam or can be seen in the patient file. For example, the expert may increase the confidence of consolidation if the patient presents with a fever (hypothetically caused by an infection which explains the consolidation as being part of an infectious process). In addition, if the patient is of young age, the change of a lung mass is further reduced - given the very low prevalence of lung cancer in the young population. One may model this non-imaging information and steer the confidence of the system in cases such as the one depicted in Figure 6. Prior knowledge may be used (e.g., lung masses are more likely in old patient than young). In the above example, with the use of auxiliary information and knowing that the patient is 28 years of age and has a fever, the chance of consolidation may be increased to 90%. Such change may be of significant impact for clinical decision making and patient triaging (e.g., avoiding unnecessary CTs).

While other types of information from the electronic health record or about general patient history is not typically used for chest radiography assessment, they can be invaluable for differential diagnosis in chest CTs. For example, conditions like Eosinophilic Pneumonia can be present with fever and cough just like COVID-19. It can be observed in images that, on CT, Eosinophilic Pneumonia presents like COVID-19 with peripheral ground-glass and consolidations, and with or without crazy paving pattern. This makes it very hard to distinguish Eosinophilic Pneumonia from the biomarkers COVID-19 using CT alone. Therefore, the present invention suggests to use the additional information from the set of sources S1, S2, S3,...Sn, DB.

In order to better distinguish it from COVID-19 in the example above, considering the following additional information is helpful:

Clinical presentation with slow onset of symptoms.

- Association with asthma;

- Eosinophilia in bronchoalveolar lavage and blood samples;

- Upper lung zone distribution.

Relevant non-imaging and imaging information for providing a result dataset, which may be used for a differential diagnosis can be made available for the radiologist by integrating the EHR systems in the radiology workflow.

In general, it is assumed that during training/inference in addition to the image I, other relevant signals S are provided, as mentioned before. These signals S are encoded as $x_1$, $x_2$, ... $x_N$, where N denotes to number of sources for non-imaging signals. For any signal $x_k$, the following properties hold:

- signal may be present or absent for a given instance/ sample.

- there is inherent uncertainty in the signal / measurement, quantified as $u(x_k)$ ∀ k (heteroscedastic aleatoric uncertainty) in terms of the evaluation task.

- $x_k$ can be differently distributed compared to any other $x_j$ (we allow categorical variables, continuous variables, and complex high dimensional signal, etc.)

In the following a robust static information fusion using deep learning models is explained in more detail.

In this scenario, the assumption is that all sources of non-imaging signal $x_1, x_2, ... x_N$ are usable (please note, the signal may still be missing due to any number of reasons). A number of techniques can be used for information fusion, including but not limited to, deep fusion models and graph neural networks.

Deep fusion models:

Mutual information

$$M(Y;\ I,\ x_1,\ x_2,\ ...\ x_N)\ \geq\ M(Y;\ I,\ x_k)\ \forall\ k,$$

where Y represents the system prediction; as such, the aim is to use all input information and exploit redundancies. Assuming noise around each signal, quantified as uncertainty $u(I); u(x_k)$, one can use methods for deep robust information fusion [6] while modeling the propagation of uncertainty through the deep model [7], e.g., using Bayesian deep learning [8]. Signal encoding architectures (e.g., variational autoencoders) can be used to compress the heterogeneous high dimensional inputs and simplify the learning process.

Graph neural models: Just as deep fusion models help in maximizing the mutual information in the selection of non-imaging signals, graph neural networks can facilitate the maximization of entropy in selecting the associations amongst the signals $x_1, x_2, ... x_N$. The non-imaging signals are connected via complex hidden underlying structures that are not always traceable. In such cases, graph neural networks can not only learn the hidden structures but can also perform prediction tasks when the structure is unavailable [10-11]. By evaluating graph entropy (ex: Von Neuman entropy, Shannon entropy), we can identify and preserve the important associations without getting lost in the complexity of these hidden structures.

Von Neuman Entropy: Assuming that all the non-imaging signals can be represented onto the same latent space, let G = (V, E, W) denote a graph with the set of vertices V ∈ {$x_1, x_2, ... x_N$} and the set of edges E, and the weight matrix W. The combinatorial graph Laplacian matrix of G is defined as:

$$L(G)\ =\ S\ -\ W,$$

where S is a diagonal matrix and its diagonal entry

$$s_i = \sum_{j=1}^{n} W_{ij}$$

The density matrix of a graph G is defined as

$$\rho_G = \frac{1}{tr(L(G))} L(G)\ ,$$

where tr is the trace of the matrix.

Thus, the entropy of the graph G is given by

$$H(G) := H(\rho_G).$$

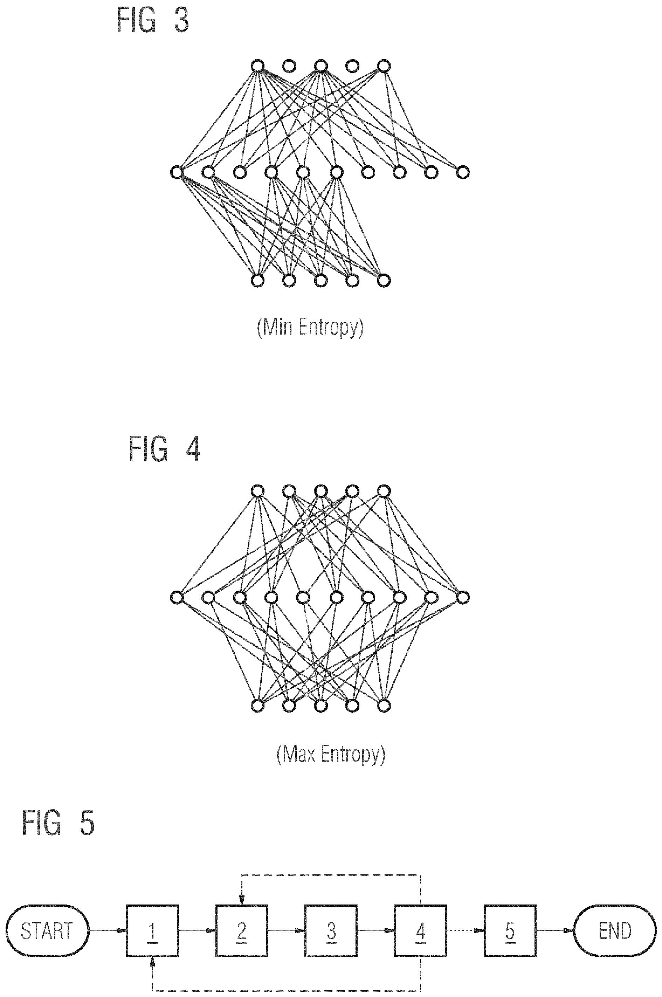

Fig. 3 and 4 show two graphs constructed with minimum and maximum entropy using a greedy algorithm to explore their properties [12]. Under the constraint of using the same number of edges or associations, the entropy of graph in Figure 3 is lesser than that of the graph in Figure 4. Almost half of the connections from the first layer to bottom layer have been blocked and several vertices are deactivated due to the minimum entropy construction in graph 3. On the contrary, a "balanced" graph that has a higher regularity tends to have a larger entropy.

In the following an information distillation process is described for optimal selection of the input sources or of (additional) information, provided by these sources using Deep Reinforcement Learning.

In the second scenario (where the entirety of the non-imaging signals is not available; it is either available partially or not at all), a subset of the non-imaging signal sources is hidden. Assume without loss of generality that only $x_1$, $x_2$, ... $x_K$ are available with K < N. In addition to the noise/uncertainty associated with each signal, we also associate a cost of acquisition or cost of measurement $c(x_k) \forall k$. One can envision this cost arising during a building stage of the training database (in the sense of cost of acquiring data from a clinical site), or during inference as a request to the user, e.g., "based on the current information the prediction is Y with high uncertainty, this uncertainty may be significantly reduced if variable $x_{K+1}$ would be available (of course each measurement/clinical test comes at a cost)".

One may formulate the problem as follows: What would be a subset of S additional sources of information (from the set $x_{k+1}$ ... $x_N$) which would minimize the cost of acquisition while optimally reducing uncertainty in the prediction? Without considering the element of cost, a potential solution is by using a feature selection strategy, equivalent to selection of signal sources, such that the uncertainty around the prediction Y is minimized [9].

One may formulate this problem in the context of reinforcement learning. Assume a decision process (DP) that is (non-) Markovian

$$M = (S, A, T, R, \eta),$$

where S denotes the state space, A the action space, T the stochastic transition process, R the reward function and $\eta$ the discount factor. The state is defined by the observable information (initially I, $x_1$, $x_2$, ... $x_K$). Actions allow for selection of additional sources from ($x_{k+1}$, ... $x_N$). The DP is non-Markovian in the sense that actions cannot be executed twice. The reward function R can be designed to minimize the cost or minimize the predictive uncertainty around the Y. Also, joint optimization is possible, i.e., minimize predictive uncertainty, while not exceeding a threshold of total cost for selection. Powering the reinforcement learning model using deep architectures would allow for the effective modeling of the complex and diverse input signal. Similar strategies may be used as described above, in the section relating to robust static information fusion using DNNs to design the learning architecture, i.e., Bayesian model, uncertainty propagation models, etc. Q-learning or actor critic strategies can be applied.

Unavailable sources of information: Using an actor critic architecture, one can also model the situation where a subset of actions that are not executable. In other words, during inference, the user can indicate if a certain subset of sources ($x_{k+1}$ ... $x_N$) can/will not be provided. In that case, the optimization model would avoid these actions.

Fig. 5 is a flow chart of a method for providing uncertainty prediction for a machine learning based result. After Start of the method, in step 1 the input data is received from the imaging and non-imaging sources. In step 2, the information fusion algorithm is provided in a storage MEM of a computer and the received input data are forwarded to this algorithm, which is executed in step 3. After execution, in step 4, the result r is provided with an uncertainty prediction of the result of the machine learning model M. Optionally, the method may branch back to step 1 for requiring more input data and/or to step 2. The latter may be the case, if an update of the image fusion algorithm and/or model may be provided, and which needs to be applied and executed on the data. This optional process steps are depicted in Fig. 5 via doted lines. Another optional step 5 is to apply or execute a selection algorithm for selecting a subset of provided input data, which minimizes a cost function and/or reduces uncertainty by using a reinforcement learning model. This improves performance as the relevant input data may be indicated and selected for being provided to the information fusion algorithm.

Generally, a single unit or device may fulfil the functions of several items recited in the claims. The mere fact that certain measures are recited in mutually different dependent claims does not indicate that a combination of these measures cannot be used to advantage.

Any reference signs in the claims should not be construed as limiting the scope.

List of references:

1. Irvin, J., Rajpurkar, P., Ko, M., Yu, Y., Ciurea-Ilcus, S., Chute, C., Marklund, H., Haghgoo, B., Ball, R., Shpanskaya, K., et al., 2019. CheXpert: A large chest radiograph dataset with uncertainty labels and expert comparison, in: AAAI Conference on Artificial Intelligence, pp. 590-597.

2. Guendel, S., Grbic, S., Georgescu, B., Liu, S., Maier, A., Comaniciu, D., 2018. Learning to recognize abnormalities in chest X-rays with location-aware dense networks, in: Iberoamerican Congress on Pattern Recognition, Springer.

pp. 757-765

3. Ghesu, F.C., Georgescu, B., Gibson, E., Guendel, S., Kalra, M.K., Singh, R., Digumarthy, S.R., Grbic, S., Comaniciu, D., 2019. Quantifying and leveraging classification uncertainty for chest radiograph assessment, in: International Conference on Medical Image Computing and Computer Assisted Intervention, Springer International Publishing. pp. 676-684.

4. Rajpurkar, P., Irvin, J., Ball, R.L., Zhu, K., Yang, B., Mehta, H., Duan, T., Ding, D., Bagul, A., Langlotz, C.P., et al., 2018. Deep learning for chest radiograph diagnosis: A retrospective comparison of the CheXNeXt algorithm to practicing radiologists. PLoS medicine 15.

5. Florin C. Ghesu, Bogdan Georgescu, Awais Mansoor, Youngjin Yoo, Eli Gibson, R.S. Vishwanath, Abishek Balachandran, James M. Balter, Yue Cao, Ramandeep Singh, Subba R. Digumarthy, Mannudeep K. Kalra, Sasa Grbic, Dorin Comaniciu, Quantifying and leveraging predictive uncertainty for medical image assessment, Medical Image Analysis, Volume 68, 2021

6. Taewan Kim, Joydeep Ghosh, On Single Source Robustness in Deep Fusion Models, NIPS 2019

7. Abdelaziz, Ahmed H., et al. "Uncertainty propagation through deep neural networks." Interspeech 2015. 2015.

8. Kendall, Alex, and Yarin Gal. "What uncertainties do we need in bayesian deep learning for computer vision?." arXiv preprint arXiv:1703.04977 (2017).

9. Zhou, Xiang Sean. et al "Conditional feature sensitivity: A unifying view on active recognition and feature selection." Proceedings Ninth IEEE International Conference on Computer Vision. IEEE, 2003.

10. Edward Choi, Zhen Xu, Yujia Li, Michael W. Dusenberry, Gerardo Flores, Yuan Xue and Andrew M. Dai. "Learning the Graphical Structure of Electronic Health Records with Graph Convolutional Transformer". Association for the Advancement of Artificial Intelligence (AAAI) (2020)

11. Edward Choi, Cao Xiao, Walter F. Stewart and Jimeng Sun. "MiME: Multilevel Medical Embedding of Electronic Health Records for Predictive Healthcare." NIPS 2018

12. Minjing Dong, Hanting Chen, Yunhe Wang, Chang Xu. "Crafting Efficient Neural Graph of Large Entropy." International Joint Conference on Artificial Intelligence (IJCAI) 2019

## Claims

1. Computer-implemented method for providing an uncertainty prediction for a medical assessment on imaging data (I), being provided by a machine-learning system (M), comprising the method steps of:

   - Receiving (1) a set of input data, comprising the imaging data (I), which have been provided to the machine-learning system (M) and non-imaging data, each represented as a signal (S) with some degree of noise, being quantified as uncertainty, in particular aleatoric uncertainty, wherein the non-imaging data comprise medical or healthcare data in a digital format or representation, which do not comprise image data acquired from an imaging modality;
   - Providing (2) an information fusion algorithm, wherein the information fusion algorithm is an algorithm for combining different data sets, provided in different formats, including the imaging data and the non-imaging data, wherein the information fusion algorithm uses an information fusion model and/or a graph neural network, being optimized for maximizing entropy in the non-imaging data;
   - Applying (3) the received set of input data on the provided information fusion algorithm, while modeling the propagation of uncertainty through the information fusion algorithm to predict an uncertainty for the medical assessment as a result (r), provided by the machine-learning system (M), based on the provided set of input data.

2. Method according to the directly preceding claim, wherein the entropy of the information fusion model and/or the graph neural network, in particular the von Neumann entropy, is optimized by a greedy algorithm.

3. Method according to any of the preceding claims, wherein the method further comprises:

- Applying (5) a selection algorithm for selecting a subset of provided input data, which minimizes a cost function and/or reduces uncertainty by using a reinforcement learning model.

4. Method according to any of the preceding claims, wherein input data of a set of input data sources (S, DB) may be present or absent and wherein the method provides a guided decision which of the absent input data sources (S, DB) would reduce uncertainty and/or minimize a cost function.

5. Method according to any of the preceding claims, wherein providing input data of the set of input data sources (S, DB) may comprise measuring and/or acquiring data from imaging modalities and/or from medical databases (DB).

6. Method according to claim 3 in combination with any of the preceding claims, wherein the reinforcement learning model is based on a decision process, in particular a non-Markovian decision process

$$M = (S, A, T, R, \eta),$$

where S denotes a state space, A an action space, T a stochastic transition process, R a reward function and $\eta$ a discount factor, wherein actions represent providing additional input data sources (S, DB).

7. Method according to the directly preceding claim, wherein the reward function is defined to minimize the cost and/or to minimize the predicted uncertainty.

8. Method according to any of the preceding claims, wherein the non-imaging data comprises biomarkers, clinical notes, image annotations, medical report dictations, measurements, laboratory values, diagnostic codes, data from an EHR-database (DB), and/or anamnestic data of the patient.

9. Method according to claim 3 in combination with any of the preceding claims, wherein an uncertainty propagation model comprising a Bayesian deep model and/or Q-Learning and/or actor critic learning is used for the reinforcement learning.

10. Method according to any of the preceding claims, wherein an uncertainty propagation model, in particular a Bayesian deep model is used in the information fusion model.

11. Method according to any of the preceding claims, wherein the information fusion model is capable of processing a situation, where a subset of input data sources (S, DB) is not available or only available by certain costs.

12. Method according to any of the preceding claims, wherein the predicted uncertainty is patient-specific, and/or imaging data specific and/or signal specific.

13. Method according to claim 6 in combination with any of the preceding claims, wherein on a user interface (UI), a set of interaction buttons is provided so that a user can indicate that an input data source (S, DB) is not available during inference or that the action space of the non-Markovian decision process is limited to the input data sources (S, DB), being available so that the user may select the type of optimization and in particular if he or she wants to minimize prediction uncertainty or costs.

14. An uncertainty quantifier for a medical assessment on imaging data, being provided by a machine-learning system (M), which is adapted to execute the method according to any of the preceding claims, comprising:

- An input interface (II) for connecting to a set of input data sources (S, DB) for receiving a set of input data, comprising the imaging data, which have been provided to the machine-learning system and non-imaging data, each represented as a signal (S) with noise, being quantified as uncertainty, in particular aleatoric uncertainty, wherein the non-imaging data comprise medical or healthcare data in a digital format or representation, which do not comprise image data acquired from an imaging modality;
- A storage (MEM) for storing an information fusion algorithm, wherein the information fusion algorithm is an algorithm for combining different data sets, provided in different formats, including the imaging data and the non-imaging data, wherein the information fusion algorithm uses an information fusion model and/or a graph neural network, being optimized for maximizing entropy in the non-imaging data;

- A processing unit (P) which is configured for applying the received set of input data on the provided information fusion algorithm while modeling the propagation of uncertainty through the information fusion algorithm to predict uncertainty of the medical assessment, which has been provided by the machine-learning system (M), based on the provided set of input data.

- An output interface (OI) for providing the predicted uncertainty as result (r).

15. Medical system for a medical assessment on imaging data, being provided by a machine-learning system (M) with a set of medical input data sources (S, DB) and with an uncertainty quantifier (Q) according to the directly preceding claim.

16. A computer program product comprising program elements which induce a computer to execute the steps of the method for providing an uncertainty prediction for a machine-learning based medical assessment on imaging data according to any of the preceding method claims, when the program elements are loaded into a memory of the computer or are executed thereon.

**Patentansprüche**

1. Computerimplementiertes Verfahren zum Bereitstellen einer Unsicherheitsvorhersage für eine medizinische Bewertung von Bildgebungsdaten (I), die durch ein maschinelles Lernsystem (M) bereitgestellt wird, umfassend die folgenden Verfahrensschritte:

   - Empfangen (1) eines Satzes von Eingabedaten, umfassend die Bildgebungsdaten (I), die dem maschinellen Lernsystem (M) bereitgestellt wurden, und Nicht-Bildgebungsdaten, die jeweils als ein Signal (S) mit einem gewissen Grad an Rauschen dargestellt werden, das als Unsicherheit, insbesondere aleatorische Unsicherheit, quantifiziert wird, wobei die Nicht-Bildgebungsdaten medizinische oder Gesundheitsdaten in einem digitalen Format oder einer digitalen Darstellung umfassen, die keine von einer Bildgebungsmodalität erfassten Bilddaten umfassen;
   - Bereitstellen (2) eines Informationsfusionsalgorithmus, wobei der Informationsfusionsalgorithmus ein Algorithmus zum Kombinieren unterschiedlicher Datensätze ist, die in unterschiedlichen Formaten bereitgestellt werden, die die Bildgebungsdaten und die Nicht-Bildgebungsdaten beinhalten, wobei der Informationsfusionsalgorithmus ein Informationsfusionsmodell und/oder ein neuronales Graphennetz verwendet, das zum Maximieren einer Entropie in den Nicht-Bildgebungsdaten optimiert ist;
   - Anwenden (3) des empfangenen Satzes von Eingabedaten auf den bereitgestellten Informationsfusionsalgorithmus während eines Modellierens der Ausbreitung von Unsicherheit durch den Informationsfusionsalgorithmus, um eine Unsicherheit für die medizinische Bewertung als ein Ergebnis (r), bereitgestellt durch das maschinelle Lernsystem (M), basierend auf dem bereitgestellten Satz von Eingabedaten vorherzusagen.

2. Verfahren nach dem direkt vorhergehenden Anspruch, wobei die Entropie des Informationsfusionsmodells und/oder des neuronalen Graphennetzes, insbesondere die Von-Neumann-Entropie, durch einen Greedy-Algorithmus optimiert wird.

3. Verfahren gemäß einem der vorhergehenden Ansprüche, wobei das Verfahren ferner Folgendes umfasst:

   - Anwenden (5) eines Auswahlalgorithmus zum Auswählen eines Teilsatzes bereitgestellter Eingabedaten, der durch Verwenden eines verstärkenden Lernmodells eine Kostenfunktion minimiert und/oder eine Unsicherheit reduziert.

4. Verfahren nach einem der vorhergehenden Ansprüche, wobei Eingabedaten eines Satzes von Eingabedatenquellen (S, DB) vorhanden oder nicht vorhanden sein können und wobei das Verfahren eine geführte Entscheidung bereitstellt, welche der nicht vorhandenen Eingabedatenquellen (S, DB) eine Unsicherheit reduzieren und/oder eine Kostenfunktion minimieren würde.

5. Verfahren nach einem der vorhergehenden Ansprüche, wobei das Bereitstellen von Eingabedaten des Satzes von Eingabedatenquellen (S, DB) Messen und/oder Erfassen von Daten von Bildgebungsmodalitäten und/oder von medizinischen Datenbanken (DB) umfassen kann.

6. Verfahren nach Anspruch 3 in Kombination mit einem der vorhergehenden Ansprüche, wobei das verstärkende Lernmodell auf einem Entscheidungsprozess, insbesondere einem nicht-markovischen Entscheidungsprozess, basiert

$$M = (S, A, T, R, \eta),$$

wobei S einen Zustandsraum, A einen Aktionsraum, T einen stochastischen Übergangsprozess, R eine Belohnungsfunktion und $\eta$ einen Diskontierungsfaktor bezeichnet, wobei Aktionen ein Bereitstellen zusätzlicher Eingabedatenquellen (S, DB) darstellen.

7. Verfahren nach dem direkt vorhergehenden Anspruch, wobei die Belohnungsfunktion so definiert wird, dass die Kosten minimiert werden und/oder die vorhergesagte Unsicherheit minimiert wird.

8. Verfahren nach einem der vorhergehenden Ansprüche, wobei die Nicht-Bildgebungsdaten Biomarker, klinische Notizen, Bildannotationen, Diktate für medizinische Berichte, Messungen, Laborwerte, Diagnosecodes, Daten aus einer ePA-Datenbank (DB) und/oder anamnestische Daten des Patienten umfassen.

9. Verfahren nach Anspruch 3 in Kombination mit einem der vorhergehenden Ansprüche, wobei für das verstärkende Lernen ein Unsicherheitsausbreitungsmodell verwendet wird, das ein Bayessches Tiefenmodell und/oder Q-Learning und/oder Actor-Critic-Lernen umfasst.

10. Verfahren nach einem der vorhergehenden Ansprüche, wobei in dem Informationsfusionsmodell ein Unsicherheitsausbreitungsmodell, insbesondere ein Bayessches Tiefenmodell, verwendet wird.

11. Verfahren nach einem der vorhergehenden Ansprüche, wobei das Informationsfusionsmodell in der Lage ist, eine Situation zu verarbeiten, in der ein Teilsatz von Eingabedatenquellen (S, DB) nicht oder nur durch bestimmte Kosten verfügbar ist.

12. Verfahren nach einem der vorhergehenden Ansprüche, wobei die vorhergesagte Unsicherheit patientenspezifisch und/oder bildgebungsdatenspezifisch und/oder signalspezifisch ist.

13. Verfahren nach Anspruch 6 in Kombination mit einem der vorhergehenden Ansprüche, wobei auf einer Benutzerschnittstelle (UI) ein Satz von Interaktionsschaltflächen bereitgestellt wird, so dass ein Benutzer angeben kann, dass eine Eingabedatenquelle (S, DB) während einer Inferenz nicht verfügbar ist oder dass der Aktionsraum des nicht-markovischen Entscheidungsprozesses auf die Eingabedatenquellen (S, DB) beschränkt ist, die verfügbar sind, so dass der Benutzer die Art von Optimierung auswählen kann und insbesondere, ob er oder sie die Vorhersageunsicherheit oder die Kosten minimieren möchte.

14. Unsicherheitsquantifikator für eine medizinische Bewertung von Bildgebungsdaten, die durch ein maschinelles Lernsystem (M) bereitgestellt wird, das dazu eingerichtet ist, das Verfahren nach einem der vorhergehenden Ansprüche auszuführen, umfassend:

   - eine Eingabeschnittstelle (II) zum Verbinden mit einem Satz von Eingabedatenquellen (S, DB) zum Empfangen eines Satzes von Eingabedaten, umfassend die Bildgebungsdaten, die dem maschinellen Lernsystem bereitgestellt wurden, und Nicht-Bildgebungsdaten, die jeweils als ein Signal (S) mit Rauschen dargestellt werden, das als Unsicherheit, insbesondere aleatorische Unsicherheit, quantifiziert wird, wobei die Nicht-Bildgebungsdaten medizinische oder Gesundheitsdaten in einem digitalen Format oder einer digitalen Darstellung umfassen, die keine von einer Bildgebungsmodalität erfassten Bilddaten umfassen;
   - einen Speicher (MEM) zum Speichern eines Informationsfusionsalgorithmus, wobei der Informationsfusionsalgorithmus ein Algorithmus zum Kombinieren unterschiedlicher Datensätze ist, die in unterschiedlichen Formaten bereitgestellt werden, die die Bildgebungsdaten und die Nicht-Bildgebungsdaten beinhalten, wobei der Informationsfusionsalgorithmus ein Informationsfusionsmodell und/oder ein neuronales Graphennetz verwendet, das zum Maximieren einer Entropie in den Nicht-Bildgebungsdaten optimiert ist;
   - eine Verarbeitungseinheit (P), die zum Anwenden des empfangenen Satzes von Eingabedaten auf den bereitgestellten Informationsfusionsalgorithmus während eines Modellierens der Ausbreitung von Unsicherheit durch den Informationsfusionsalgorithmus ausgelegt ist, um eine Unsicherheit der medizinischen Bewertung, die durch das maschinelle Lernsystem (M) bereitgestellt wurde, basierend auf dem bereitgestellten Satz von Eingabedaten vorherzusagen.
- eine Ausgabeschnittstelle (OI) zum Bereitstellen der vorhergesagten Unsicherheit als Ergebnis (r).

**15.** Medizinisches System für eine medizinische Bewertung von Bildgebungsdaten, die durch ein maschinelles Lernsystem (M) mit einem Satz von medizinischen Eingabedatenquellen (S, DB) und mit einem Unsicherheitsquantifikator (Q) nach dem direkt vorhergehenden Anspruch bereitgestellt wird.

**16.** Computerprogrammprodukt, umfassend Programmelemente, die einen Computer dazu veranlassen, die Schritte des Verfahrens zum Bereitstellen einer Unsicherheitsvorhersage für eine auf maschinellem Lernen basierte medizinische Bewertung von Bildgebungsdaten nach einem der vorhergehenden Verfahrensschritte auszuführen, wenn die Programmelemente in einen Speicher des Computers geladen oder darauf ausgeführt werden.

## Revendications

**1.** Procédé mis en œuvre par ordinateur pour fournir une prédiction d'incertitude pour une évaluation médicale sur des données d'imagerie (I), étant fournie par un système d'apprentissage automatique (M), comprenant les étapes de procédé de :

- Réception (1) d'un ensemble de données d'entrée, comprenant les données d'imagerie (I), qui ont été fournies au système d'apprentissage automatique (M) et des données de non-imagerie, représentées chacune comme un signal (S) avec un certain degré de bruit, étant quantifié comme une incertitude, en particulier une incertitude aléatoire, les données de non-imagerie comprenant des données médicales ou de soins sous format ou représentation numérique, qui ne comprennent pas de données d'images acquises en provenance d'une modalité d'imagerie ;
- Fourniture (2) d'un algorithme de fusion d'informations, l'algorithme de fusion d'informations étant un algorithme pour la combinaison de différents ensembles de données, fournis dans des formats différents, dont les données d'imagerie et les données de non imagerie, l'algorithme de fusion d'informations utilisant un modèle de fusion d'informations et/ou un réseau de neurones graphique, étant optimisé pour maximiser l'entropie dans les données de non imagerie ;
- Application (3) de l'ensemble reçu de données d'entrée sur l'algorithme de fusion d'informations fourni, en modélisant la propagation d'incertitude à travers l'algorithme de fusion d'informations pour prédire une incertitude pour l'évaluation médicale en tant que résultat (R), fournie par le système d'apprentissage automatique (M), sur la base de l'ensemble fourni de données d'entrée.

**2.** Procédé selon la revendication directement précédente, dans lequel l'entropie du modèle de fusion d'informations et/ou du réseau de neurones graphique, notamment l'entropie de Von Neumann, est optimisée par un algorithme glouton.

**3.** Procédé selon de quelconques des revendications précédentes, dans lequel le procédé comprend en outre :

- L'application (5) d'un algorithme de sélection pour sélectionner un sous-ensemble de données d'entrée fournies, qui minimise une fonction de coût et/ou réduit une incertitude en utilisant un modèle d'apprentissage par renforcement.

**4.** Procédé selon l'une quelconque des revendications précédentes, dans lequel des données d'entrée d'un ensemble de sources de données d'entrée (S, DB) peuvent être présentes ou absentes et dans lequel le procédé fournit une décision guidée concernant les sources de données d'entrée absentes (S, DB) qui réduiraient une incertitude et/ou minimiseraient une fonction de coût.

**5.** Procédé selon l'une quelconque des revendications précédentes, dans lequel la fourniture de données d'entrée de l'ensemble de sources de données d'entrée (S, DB) peut comprendre la mesure et/ou l'acquisition de données en provenance de modalités d'imagerie et/ou en provenance de bases de données (DB) médicales.

**6.** Procédé selon la revendication 3 en combinaison avec l'une quelconque des revendications précédentes, dans lequel le modèle d'apprentissage par renforcement est basé sur un processus de décision, en particulier un processus de décision non markovien

$$M = (S, A, T, R, \eta),$$

où S désigne un espace d'état, A un espace d'action, T un processus de transition stochastique, R une fonction de récompense et $\eta$ un facteur de remise, dans lequel des actions représentent la fourniture de sources de données d'entrée (S, DB) supplémentaires.

7. Procédé selon la revendication directement précédente, dans lequel la fonction de récompense est définie pour minimiser le coût et/ou pour minimiser l'incertitude prédite.

8. Procédé selon l'une quelconque des revendications précédentes, dans lequel les données de non-imagerie comprennent des biomarqueurs, des notes cliniques, des annotations d'image, des dictées de rapport médical, des mesures, des valeurs laboratoires, des codes de diagnostic, des données provenant d'une base de données (DB) EHR et/ou données anamnestiques du patient.

9. Procédé selon la revendication 3 en combinaison avec l'une quelconque des revendications précédentes, dans lequel un modèle de propagation d'incertitude comprenant un modèle profond bayésien et/ou un apprentissage Q-Learning et/ou un apprentissage acteur-critique est utilisé pour l'apprentissage par renforcement.

10. Procédé selon l'une quelconque des revendications précédentes, dans lequel un modèle de propagation d'incertitude, en particulier un modèle profond bayésien, est utilisé dans le modèle de fusion d'informations.

11. Procédé selon l'une quelconque des revendications précédentes, dans lequel le modèle de fusion d'informations est capable de traiter une situation, dans laquelle un sous-ensemble de sources de données d'entrée (S, DB) n'est pas disponible ou seulement disponible par certains coûts.

12. Procédé selon l'une quelconque des revendications précédentes, dans lequel l'incertitude prédite est spécifique au patient et/ou spécifique à des données d'imagerie et/ou spécifique au signal.

13. Procédé selon la revendication 6 en combinaison avec l'une quelconque des revendications précédentes, dans lequel sur une interface utilisateur (UI), un ensemble de boutons d'interaction est prévu de sorte qu'un utilisateur peut indiquer qu'une source de données d'entrée (S, DB) n'est pas disponible pendant une inférence ou que l'espace d'action du processus de décision non markovien est limité aux sources de données d'entrée (S, DB), disponibles pour que l'utilisateur puisse sélectionner le type d'optimisation et en particulier s'il ou elle souhaite minimiser une incertitude prédite ou des coûts.

14. Quantificateur d'incertitude pour une évaluation médicale sur des données d'imagerie, fourni par un système d'apprentissage automatique (M), qui est conçu pour exécuter le procédé selon l'une quelconque des revendications précédentes, comprenant :

- Une interface d'entrée (II) pour une connexion à un ensemble de sources de données d'entrée (S, DB) pour recevoir un ensemble de données d'entrée, comprenant les données d'imagerie, qui ont été fournies au système d'apprentissage automatique et des données de non-imagerie, représentées chacune comme un signal (S) avec du bruit, quantifié comme une incertitude, en particulier une incertitude aléatoire, les données de non-imagerie comprenant des données médicales ou de santé sous format ou représentation numérique, qui ne comprennent pas des données d'images acquises en provenance d'une modalité d'imagerie ;
- Un stockage (MEM) pour le stockage d'un algorithme de fusion d'informations, l'algorithme de fusion d'informations étant un algorithme de combinaison de différents ensembles de données, fournis dans des formats différents, y compris les données d'imagerie et les données de non-image, l'algorithme de fusion d'informations utilisant un modèle de fusion d'informations et/ou un réseau de neurones graphique, optimisé pour maximiser l'entropie dans les données de non-image ;
- Une unité de traitement (P) qui est configurée pour appliquer l'ensemble reçu de données d'entrée sur l'algorithme de fusion d'informations fourni tout en modélisant la propagation d'incertitude à travers l'algorithme de fusion d'informations pour prédire une incertitude de l'évaluation médicale, qui a été fournie par le système d'apprentissage automatique (M), sur la base de l'ensemble fourni de données d'entrée.
- Une interface de sortie (OI) pour fournir l'incertitude prédite comme résultat (R).

15. Système médical pour une évaluation médicale de données d'imagerie, fournies par un système d'apprentissage automatique (M) avec un ensemble de sources de données d'entrée (S, DB) médicales et avec un quantificateur d'incertitude (Q) selon la revendication directement précédente.

16. Produit de programme informatique comprenant des éléments de programme qui entraînent un ordinateur à exécuter les étapes du procédé de fourniture d'une prédiction d'incertitude pour un apprentissage automatique sur la base d'une évaluation médicale sur des données d'imageries selon l'une des revendications de procédé précédentes, lorsque les éléments de programme sont chargés dans une mémoire de l'ordinateur ou sont exécutés sur celui-ci.

# FIG 1
( prior art )

```
      I
      |
   ┌──────┐        ┌──────┐        ╭──────╮
   │ CXR  │───────▶│  M   │───────▶│  r'  │
   └──────┘        └──────┘        ╰──────╯
```

# FIG 2

```
                                        ┌────┐
                                        │ Q  │
   I──┌──────────┐                       │    │
      │   CXR    │──────────────┐        │    │
      └──────────┘              │        │    │
   S1─┌──────────┐              │        │    │
      │ biomarker│──────────────┤        │    │              UI
      └──────────┘              │        │    │               |
   S2─┌──────────┐              │  ┌──┐  │    │            ┌──────┐
      │  report  │──────────────┤  │  │  │    │            │ ╭──╮ │
      └──────────┘             II  │ P│ OI────────────────▶│ │r │ │
   S3─┌──────────┐              │  │  │  │    │            │ ╰──╯ │
      │lab values│──────────────┤  └──┘  │    │            └──────┘
      └──────────┘              │        │    │
   S4─┌──────────┐              │        │    │
      │physiolog.│──────────────┤        │    │
      │measurem. │              │        │    │
      └──────────┘              │        │    │
   DB─┌──────────┐              │        │    │
      │   EHR    │──────────────┘        │    │
      └──────────┘                       └────┘
         ⋮
```

## FIG 3

(Min Entropy)

## FIG 4

(Max Entropy)

## FIG 5

FIG 6

EP 4 141 886 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20190122073 A1

**Non-patent literature cited in the description**

- **KINGMA, D. P.** ; **WELLING, M.** *Auto-encoding variational bayes*, 2013
- **IRVIN, J.** ; **RAJPURKAR, P.** ; **KO, M** ; **YU, Y** ; **CIUREA-ILCUS, S** ; **CHUTE, C** ; **MARKLUND, H.** ; **HAGHGOO, B.** ; **BALL, R** ; **SHPANSKAYA, K. et al.** CheXpert: A large chest radiograph dataset with uncertainty labels and expert comparison. *AAAI Conference on Artificial Intelligence*, 2019, 590-597
- Learning to recognize abnormalities in chest X-rays with location-aware dense networks. **GUENDEL, S.** ; **GRBIC, S** ; **GEORGESCU, B** ; **LIU, S** ; **MAIER, A** ; **COMANICIU, D.** Iberoamerican Congress on Pattern Recognition. Springer, 2018, 757-765
- Quantifying and leveraging classification uncertainty for chest radiograph assessment. **GHESU, F.C** ; **GEORGESCU, B** ; **GIBSON, E.** ; **GUENDEL, S** ; **KALRA, M.K** ; **SINGH, R** ; **DIGUMARTHY, S.R** ; **GRBIC, S** ; **COMANICIU, D**. International Conference on Medical Image Computing and Computer Assisted Intervention. Springer International Publishing, 2019, 676-684
- **RAJPURKAR, P** ; **IRVIN, J** ; **BALL, R.L.** ; **ZHU, K** ; **YANG, B** ; **MEHTA, H.** ; **DUAN, T** ; **DING, D** ; **BAGUL, A** ; **LANGLOTZ, C.P et al.** Deep learning for chest radiograph diagnosis: A retrospective comparison of the CheXNeXt algorithm to practicing radiologists. *PLoS medicine*, 2018, vol. 15
- **FLORIN C. GHESU** ; **BOGDAN GEORGESCU** ; **AWAIS MANSOOR** ; **YOUNGJIN YOO** ; **ELI GIBSON** ; **R.S. VISHWANATH** ; **ABISHEK BALACHANDRAN** ; **JAMES M. BALTER** ; **YUE CAO** ; **RAMANDEEP SINGH**. Quantifying and leveraging predictive uncertainty for medical image assessment. *Medical Image Analysis*, 2021, vol. 68
- **TAEWAN KIM** ; **JOYDEEP GHOSH**. On Single Source Robustness in Deep Fusion Models. *NIPS*, 2019
- **ABDELAZIZ** ; **AHMED H. et al.** Uncertainty propagation through deep neural networks.. *Interspeech 2015*, 2015
- **KENDALL, ALEX** ; **YARIN GAL**. What uncertainties do we need in bayesian deep learning for computer vision?. *arXiv preprint arXiv:1703.04977*, 2017
- Conditional feature sensitivity: A unifying view on active recognition and feature selection. **ZHOU, XIANG SEAN et al.** Proceedings Ninth IEEE International Conference on Computer Vision. IEEE, 2003
- **EDWARD CHOI** ; **ZHEN XU** ; **YUJIA LI** ; **MICHAEL W. DUSENBERRY** ; **GERARDO FLORES** ; **YUAN XUE** ; **ANDREW M. DAI**. Learning the Graphical Structure of Electronic Health Records with Graph Convolutional Transformer. *Association for the Advancement of Artificial Intelligence (AAAI)*, 2020
- **EDWARD CHOI** ; **CAO XIAO** ; **WALTER F. STEWART** ; **JIMENG SUN**. MiME: Multilevel Medical Embedding of Electronic Health Records for Predictive Healthcare. *NIPS*, 2018
- **MINJING DONG** ; **HANTING CHEN** ; **YUNHE WANG** ; **CHANG XU**. Crafting Efficient Neural Graph of Large Entropy. *International Joint Conference on Artificial Intelligence (IJCAI)*, 2019